Europäisches Patentamt

European Patent Office

Office européen des brevets

(19)

(11)  EP 1 205 748 A1

(12)  EUROPEAN PATENT APPLICATION

(43) Date of publication:
15.05.2002 Bulletin 2002/20

(51) Int Cl.7: G01N 29/02

(21) Application number: 01119945.2

(22) Date of filing: 17.08.2001

(84) Designated Contracting States:
AT BE CH CY DE DK ES FI FR GB GR IE IT LI LU
MC NL PT SE TR
Designated Extension States:
AL LT LV MK RO SI

(30) Priority: 13.11.2000 SE 0004140

(71) Applicant: Siemens-Elema AB
171 95 Solna 1 (SE)

(72) Inventors:
• Skog, Göran
  168 56 Bromma (SE)
• Ahlmen, Christer
  192 51 Sollentuna (SE)
• Östberg, Anders
  171 55 Solna (SE)

(54)  **Acoustic gas analyser**

(57)    A measurement cell (2) is operably connectable to analysis means (24,26,30) adapted to determine compositional information of a gas dependent on the analysis of acoustic velocities. The cell (2) comprises a sample chamber (4) for receiving a gas mixture to be analysed and a reference chamber (6) for receiving a known reference gas, separated by a common wall section (8) at least part of which (10) can communicate pressure changes and preferably at least part of which (12) can communicate humidity changes between the chambers (4;6). An acoustic transmitter/receiver arrangement (18a,20a;18b,20b) is operable to transmit a first acoustic signal through a gas mixture within the sample chamber (4), a second acoustic signal through reference gas within the reference chamber (6) and to receive the first and second transmitted signals for use in determining acoustic velocities.

Fig. 1

EP 1 205 748 A1

## Description

The present invention relates to an acoustic gas analyser for the analysis of relative proportions of gases in gas mixtures and in particular to one in which a reference chamber containing a known reference gas is employed for calibration.

In medical and clinical settings it is useful to be able to measure accurately the concentrations of respiratory (inspiration and/or expiration) gases or changes therein since these can provide valuable information on patient metabolic conditions. This is particularly the case during the provision of mechanical respiratory aid to a patient where knowledge of the relative and absolute amounts of oxygen and carbon dioxide within the expiration gas may be used to provide information on the metabolisation of oxygen as well as respiratory function. Moreover, knowledge of the oxygen/nitrogen ratio in an inspiration gas is useful for controlling or monitoring the provision of mechanical respiratory aid.

A gas analyser is known, for example from WO 92/03724, for acoustically analysing the ratios of a mixture of gases comprising two known gases, such as the oxygen/nitrogen ratio in a breathing gas to be supplied to a patient from which the oxygen concentration or changes therein can be determined. The known analyser utilises the physical phenomenon that acoustic waves travel with different velocities through different gases. The velocity of sound, V, through a gas is known to be proportional to $(T/M)^{0.5}$ where M is the molecular weight of the gas and T is its absolute temperature. Thus for a gas mixture at a known temperature monitoring the velocity of sound, V, provides a measure of the relative concentrations of the constituents of the gas.

The known analyser comprises an ultrasonic transducer arrangement adapted to transmit ultrasound pulses through a sample gas mixture within a measurement cell and to receive the so transmitted pulses; a temperature sensor to monitor the gas temperature within the cell; and a calculations unit for calculating the transit time of the ultrasound pulses and for determining the ratio of the gases therefrom.

However, in the above described analyser temperature dependent mechanical effects on the operation of the transducer and other non-linear temperature variations are not catered for.

An improvement to the analyser described above is disclosed in US 5,581,014 in which non-linear and mechanical effects due to temperature changes are compensated for automatically. The improved analyser comprises a measurement cell having a sample chamber, into which a sample gas is drawn, and a physically separate reference chamber which holds a reference gas of known composition; an acoustic generator for simultaneously generating an identical acoustic signal within the sample and the reference chambers; an acoustic receiver for separately receiving the generated acoustic signals after their transmission through gas within respective sample and reference chambers; and a signal analyser which calculates the velocity of sound through gas within the sample chamber using a clock signal generated by the acoustic signal received after transmission through the gas within the reference chamber. Thus, provided that the temperatures of the gas within the reference chamber and the sample chamber are allowed to equilibrate then any temperature induced effects may be compensated for.

However, changes in the temperature of the sample gas which occur faster than the temperatures can equilibrate will remain uncompensated for.

Particularly, the temperature of the sample gas will vary according to its pressure so that in circumstances where the sample gas has a variable and rapidly changing pressure, such as typically found in inspiration and expiration gases during mechanical respiratory aid, inaccuracies in the measured gas ratio can occur.

Additionally, density variations of the sample gas due to an unknown humidity level can also be a problem. This is particularly problematical if the expiration gas of a patient is being monitored but may also be a problem if the inspiration gas is being monitored where the gas is often humidified to ambient humidity levels.

It is an aim of the present invention to provide an acoustic gas analyser in which compensation of acoustic velocity measurements in a sample gas mixture for pressure and/or humidity effects may be achieved.

Accordingly, the present invention provides a measurement cell for use in an acoustic gas analyser as described in and characterised by the present Claim 1. Having a cell in which acoustic velocities in both a sample chamber and a reference chamber may be measured and in which the reference chamber and the sample chamber are arranged separated by a common wall, at least part of which is moveable, for example by forming it from an elastically deformable gas-tight membrane, in response to relative gas pressure changes in the sample chamber and the reference chamber then respective pressure changes of the gas mixture can be compensated for.

In this way equal pressure changes within the two chambers will occur and any pressure induced temperature effects on the acoustic velocity through gas within the sample chamber will be simply and rapidly compensated for through similar pressure induced temperature effects on the acoustic velocity through gas within the reference chamber.

Usefully, one or more water vapour transfer elements which selectively provide for the transfer of water vapour there through may be disposed within one or both the sample chamber and the reference chamber, and preferably in the common wall section, to ensure that the humidity of the gases within each chamber is the same.

Exemplary embodiments of the present invention will now be described with reference to the drawings of the accompanying figures, of which:

Fig. 1 shows a schematic block diagram of an analyser according to the present invention.

Fig. 2 show a schematic illustration of an alternative measurement cell usable in the analyser of Fig.1

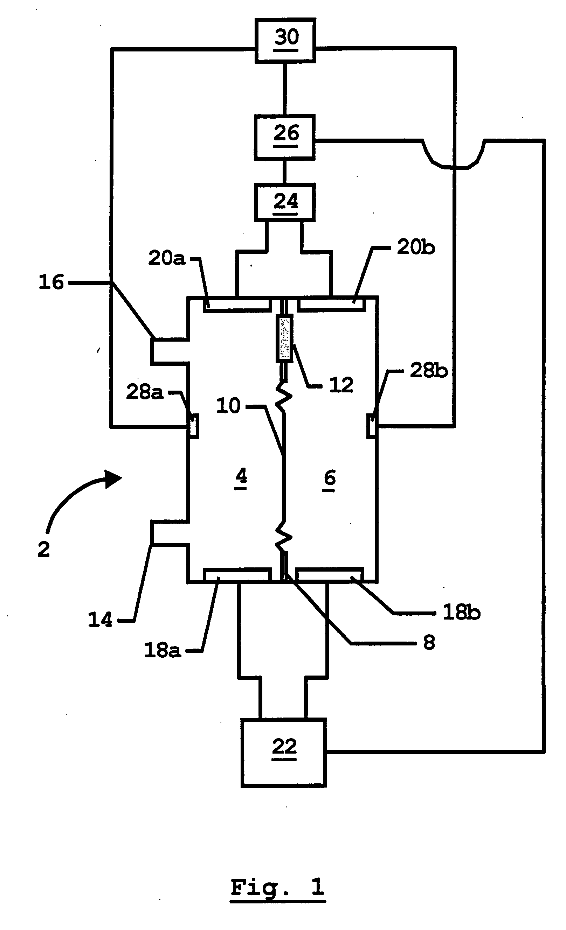

Considering now the analyser illustrated in Fig. 1, in which a measurement cell 2 is shown to comprise a sample chamber 4 and a reference chamber 6 connected by a common wall 8 which separates the chambers 4,6. By providing the sample chamber 4 and the reference chamber 6 in intimate contact and with substantially similar physical dimensions then both chambers 4,6 will be effected by ambient and other temperature changes in the same way. Sections of the wall 8 are formed of a gas-tight flexible membrane 10 and of a water vapour transfer element 12, for example formed from an ion exchange polymer membrane such as NAFION® (commercially available from E.I. DuPont de Nemours and Company, Wilmington, Delaware).

An inlet 14 and an outlet 16 are provided in the sample chamber 4 to permit a sample of a gas mixture which comprises two known gases in an unknown ratio to enter and exit. A reference gas of known composition, such as air, is held within the reference chamber 6. The flexible membrane 10 is moveable under the influence of pressure changes within gas in the sample chamber 4 to communicate the pressure change to the reference gas within the reference chamber 6 and the transfer element 12 permits only the transfer of water vapour between the chambers 4,6 to equilibrate the humidity of the gases contained therein. In this manner pressure and humidity effects on the analysis of the sample gas may be compensated for.

The analyser also comprises an acoustic transmitter/receiver arrangement for transmitting and receiving acoustic signals through gas within each of the sample chamber 4 and the reference chamber 6 and for determining acoustic velocities therefrom in a manner conventional in the art. In the present embodiment the arrangement is shown to comprise two ultrasonic transducers 18 a,b which are adapted to act as ultrasound emitters and are disposed within the sample chamber 4 and the reference chamber 6 respectively. Two further ultrasonic transducers 20 a,b are adapted to act ultrasound receivers and are also disposed within the sample chamber 4 and the reference chamber 6 respectively to receive ultrasound pulses emitted from their associated ultrasound emitters 18 a,b after passage through gas within their respective chambers 4,6. A driver unit 22 is operably connected to the two ultrasound transmitters 18 a,b to provide an electric drive pulse to the transmitters 18 a,b in order to stimulate the generation of a corresponding ultrasound pulse. On receipt of the respective ultrasound pulse the two ultrasound receivers 20 a,b generate an output electrical signal representative of the received pulse which is output to a zero-crossing detector 24.

When a zero crossing of the output electrical signal is detected as originating from a correct ultrasonic pulse the zero-crossing detector generates an output signal for use by a transit-time calculator 26. As is known in the art, the transit time calculator 26 essentially comprises a counter (not shown) which is incremented at a known clock speed. The counter is started upon receipt of a signal from the driver unit 22 which is generated simultaneously with the provision of the electric drive pulse. The counter is stopped upon receipt of the output signal from the zero-crossing detector 24. The velocity of the ultrasound pulses through gas within the respective chambers 4,6 is then determined within the transit time calculator 26 from knowledge of the counter reading, the incrementing clock speed and the separation of the emitter/receiver 18a, 20a; 18b, 20b in the sample chamber 4 and reference chamber 6 respectively.

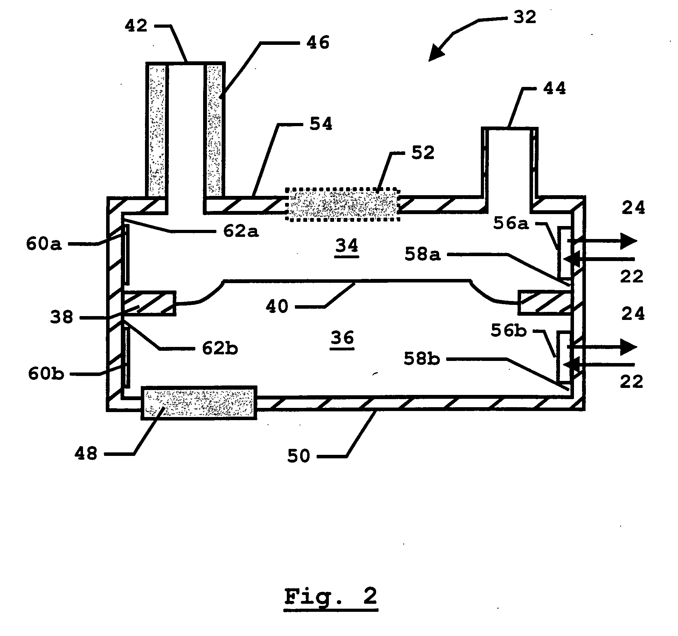

It will be appreciated that the acoustic transmitter/receiver arrangement described above may be substituted for any known arrangement for the determination of acoustic velocities, such as one which operates by determining phase changes; or one in which the transceivers 18a, 20a in at least the sample chamber 4 are configured to each operate alternately as an ultrasound emitter and an ultrasound receiver so that velocities in opposing directions of travel may be determined and used to compensate for the velocity of sample gas moving through the sample chamber 4; or the arrangement shown in Fig.2.

Thermal sensors 28a,b are disposed to monitor average gas temperatures within the sample chamber 4 and the reference chamber 6 respectively. Pressure induced changes to the gas temperatures in each chamber 4,6 occur substantially instantaneously and will not be detected by the sensors 28a,b which have response times in the region of 1 to 10 seconds.

These rapid pressure induced changes will be compensated for through movements of the membrane 10 which equalise pressure changes in the two chambers 4,6. Indeed, these temperature sensors 28a,b may be omitted where the analyser is to be used in circumstances where the average temperature of the two chambers 4,6 can be assumed to be the same. This will be the case where the two chambers 4,6 are maintained in good thermal contact.

The so determined ultrasound velocities within each of the sample chamber 4 and reference chamber 6 are output to a calculations unit 30 where they are employed, together with time averaged temperature readings from thermal sensors 28a,b (if provided), to calculate the ratio of two known gases within the gas mixture in the sample cell using the known relationship:

$$V = K \, (T/M)^{0.5} \qquad (1)$$

where

V is the acoustic velocity through gas within the relevant chamber 4,6;

T is the absolute temperature of gas within the relevant chamber 4,6;

M is the molecular weight of gas with the relevant chamber 4,6; and

K is a constant for the relevant chamber 4,6 dependent on the specific heat capacities of the gases.

Since M is known for the reference gas then a measurement of V within the reference chamber 6 provides a measure of T. Thus, since T is known, a measurement of V within the sample chamber provides a measure of M of the sample gas.

An alternative embodiment of a measurement cell according to the present invention which may substitute for the measurement cell 2 of Fig 1 is illustrated in Fig. 2.

With reference to Fig. 2, a measurement cell 32 comprises a sample chamber 34 and a reference chamber 36 which are configured with a common wall 38. A section of the common wall 38 is provided as a flexible membrane 40 which moves under pressure changes in the sample cell 34 in a manner equivalent to that of membrane 10 of Fig. 1. An inlet 42 and an outlet 44 are provided connected to the sample chamber 34. The inlet 42 is formed, at least in part, from a suitable ion exchange polymer 46 such as NAFION®, to allow equilibration of humidity between an incoming sample gas and the surrounding air. A water vapour transfer element 48, preferably formed of the same ion exchange polymer as the section 46 of the inlet 42, is provided in an outer wall 50 of the reference chamber to allow equilibration of humidity between a reference gas within the reference chamber 36 and the surrounding air. In this manner the element 48 and the section 46 provide for humidity communication between gas within the sample chamber 34 and gas within the reference chamber 36. As an alternative to forming the inlet 42 from a suitable ion exchange polymer 46 a water vapour transfer element 52 (broken lines in Fig. 2) may be placed in an outer wall 54 of the sample chamber 34 to permit humidity transfer between internal and external the sample chamber 34.

An acoustic transmitter/receiver arrangement is shown in the embodiment of Fig. 2 which comprises two acoustic transceivers 56a,b. One transceiver 56a is disposed against an end wall 58a of the sample chamber 34 and the other transceiver 56b is disposed against an end wall 58b of the reference chamber 36.

Acoustic reflectors 60a,b are disposed against opposite end walls 62a,b of respectively the sample chamber 34 and the reference chamber 36 so as to reflect towards the corresponding transceiver 56a,b incident acoustic energy emitted by that transceiver 56a,b.

These reflectors 62a,b may, of course, be omitted if the end walls 62a,b are sufficiently reflective. The transceivers 56a,b may be operably connected to a suitably modified driver unit 22 and zero crossing detector 24 of Fig. 1, as illustrated by the arrows in Fig.2. Any modification to these devices 22, 24 will be well within the scope of a person skilled in the art to make.

It will be appreciated by a person skilled in the art that an acoustic transmitter/receiver arrangement of known form, such as for example that arrangement illustrated in the embodiment of Fig.1, may readily substitute for the arrangement described above with respect to the measurement cell 32 of Fig. 2.

## Claims

1. A measurement cell (2;32) for an acoustic gas analyser comprising:

   a sample chamber (4;34) for receiving a gas mixture to be analysed;
   a reference chamber (6;36) for receiving a known reference gas;
   an acoustic transmitter and receiver arrangement (18a,20a;18b,20b;56a,60a;56b,60b) operable to transmit a first acoustic signal through a gas mixture within the sample chamber (4; 34), a second acoustic signal through reference gas within the reference chamber (6;36) and to receive the first and second transmitted signals;

   **characterised in that**
   the sample chamber (4;34) and the reference chamber (6;36) are configured with a common wall section (8;38) at least part of which (10;40) is moveable to transmit a change in gas pressure between internal the sample chamber (4;32) and internal the reference chamber (6;36).

2. A measurement cell (2) as claimed in Claim 1 **characterised in that** there is further provided a temperature monitor (28a,b) adapted to provide a measure of a time averaged temperature in each of the sample chamber (4) and the reference chamber (6).

3. A measurement cell (2) as claimed in Claim 1 or Claim 2 **characterised in that** the sample chamber (4) and the reference chamber (6) are configured with the common wall section (8) at least part of which comprises a water vapour transfer element (12) for communicating water vapour between internal the sample chamber (4) and internal the reference chamber (6).

4. A measurement cell (32) as claimed in any of the Claims 1 to 3 **characterised in that** the reference chamber (36) is provided with a water vapour transfer element (48) for communicating water vapour between internal the chamber (36) and ambient atmosphere.

5. A measurement cell (32) as claimed in Claim 4 **characterised in that** the sample chamber (34) is provided with a water vapour transfer element (46; 52) for communicating water vapour between internal the chamber (34) and ambient atmosphere.

6. An acoustic gas analyser comprising:

a measurement cell (2;32) as claimed in any preceding claim; a drive unit (22) operably connected to the transmitter (18a,b;56a,b) of the acoustic transmitter and receiver arrangement (18a,20a;18b,20b;56a,60a;56b,60b) to control the transmission thereby of acoustic energy; detector circuitry (24,26) operably connected to the receiver (20a,b;56a,b) to receive an electrical signal therefrom dependent on receipt of a transmitted acoustic signal thereby and to determine an acoustic velocity within each of the respective sample chamber (4;34) and reference chamber (6;36); and

an analyser unit (30) operably connected to the detector circuitry (24,26) to receive the determined acoustic velocities and adapted to determine compositional properties of gas within the sample chamber (4;34) therefrom.

**Fig. 1**

EP 1 205 748 A1

Fig. 2

7

**European Patent Office**

## EUROPEAN SEARCH REPORT

Application Number

EP 01 11 9945

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 3 596 510 A (SIEGEL BERNARD ET AL) 3 August 1971 (1971-08-03) * figure 1 * | 1-6 | G01N29/02 |
| X | SU 1 054 683 A (ZALKIND LEONID A;KOZMIN ALEKSANDR S; KUROCHKIN YURIJ S; KHOBOTOVA OLGA) 15 November 1983 (1983-11-15) * abstract * | 1-6 | |
| X | US 5 386 714 A (DAMES ANDREW N) 7 February 1995 (1995-02-07) * figure 1 * | 1-6 | |
| X | US 5 349 852 A (ARNOLD FINN ET AL) 27 September 1994 (1994-09-27) * column 2, line 1-10; figure 3 * * column 6 * | 1-6 | |
| A | GB 727 891 A (JOHN HUGH DAVEY WALTON) 13 April 1955 (1955-04-13) * page 3, line 100 - line 110 * * page 6, line 1 - line 12 * | 1-5 | TECHNICAL FIELDS SEARCHED (Int.Cl.7) G01N |
| A | US 3 981 176 A (JACOBS JOHN E) 21 September 1976 (1976-09-21) * column 3, line 35 - line 55 * * column 4, line 35 - line 55 * | 1-6 | |
| A | PATENT ABSTRACTS OF JAPAN vol. 006, no. 018 (P-100), 2 February 1982 (1982-02-02) & JP 56 140249 A (OSAKA GAS CO LTD), 2 November 1981 (1981-11-02) * abstract; figure 2 * | 1-6 | |
| A | DE 31 25 078 A (SCHAFFER DETLEF;KRAUSCH BERNHARD) 13 January 1983 (1983-01-13) * page 6; figures 1,2 * | 1-6 | |

-/--

The present search report has been drawn up for all claims

| Place of search | Date of completion of the search | Examiner |
|---|---|---|
| MUNICH | 25 February 2002 | Mason, W |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

## EUROPEAN SEARCH REPORT

Application Number

EP 01 11 9945

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | GB 2 087 559 A (NAT MARITIME INST) 26 May 1982 (1982-05-26) * page 2, line 60 - line 95; figure 3 * | 1-6 | |
| A | US 3 357 245 A (WOLFRUM RICHARD L) 12 December 1967 (1967-12-12) * column 4; figure 1 * | 1-6 | |
| A | EP 0 486 264 A (HUGHES AIRCRAFT CO) 20 May 1992 (1992-05-20) * column 5-7; figure 1 * | 1-6 | |
| A | GB 574 819 A (BENDIX AVIAT CORP) 22 January 1946 (1946-01-22) * figures 4,10 * | 1-6 | |

TECHNICAL FIELDS SEARCHED (Int.Cl.7)

The present search report has been drawn up for all claims

| Place of search | Date of completion of the search | Examiner |
|---|---|---|
| MUNICH | 25 February 2002 | Mason, W |

EPO FORM 1503 03.82 (P04C01)

**ANNEX TO THE EUROPEAN SEARCH REPORT
ON EUROPEAN PATENT APPLICATION NO.**

EP 01 11 9945

This annex lists the patent family members relating to the patent documents cited in the above-mentioned European search report.
The members are as contained in the European Patent Office EDP file on
The European Patent Office is in no way liable for these particulars which are merely given for the purpose of information.

25-02-2002

| Patent document cited in search report | | Publication date | Patent family member(s) | | Publication date |
|---|---|---|---|---|---|
| US 3596510 | A | 03-08-1971 | NONE | | |
| SU 1054683 | A | 15-11-1983 | SU | 1054683 A1 | 15-11-1983 |
| US 5386714 | A | 07-02-1995 | GB | 2251308 A | 01-07-1992 |
| | | | CA | 2098925 A1 | 23-06-1992 |
| | | | EP | 0563078 A1 | 06-10-1993 |
| | | | WO | 9211532 A1 | 09-07-1992 |
| US 5349852 | A | 27-09-1994 | US | 4808161 A | 28-02-1989 |
| | | | US | 4826482 A | 02-05-1989 |
| | | | US | 4976162 A | 11-12-1990 |
| | | | US | 5088515 A | 18-02-1992 |
| | | | US | 4778451 A | 18-10-1988 |
| | | | US | 5533389 A | 09-07-1996 |
| | | | US | 5526844 A | 18-06-1996 |
| | | | US | 5713865 A | 03-02-1998 |
| | | | US | 5575310 A | 19-11-1996 |
| | | | US | 5222946 A | 29-06-1993 |
| | | | US | 5935105 A | 10-08-1999 |
| | | | US | 5364371 A | 15-11-1994 |
| | | | US | 5401342 A | 28-03-1995 |
| | | | US | 5193990 A | 16-03-1993 |
| | | | US | 5178182 A | 12-01-1993 |
| | | | US | 5211201 A | 18-05-1993 |
| | | | US | 5353837 A | 11-10-1994 |
| | | | US | 5195986 A | 23-03-1993 |
| | | | US | 5241985 A | 07-09-1993 |
| | | | CA | 1290211 A1 | 08-10-1991 |
| | | | DE | 3882966 D1 | 09-09-1993 |
| | | | DE | 3882966 T2 | 25-11-1993 |
| | | | EP | 0332690 A1 | 20-09-1989 |
| | | | JP | 2501198 T | 26-04-1990 |
| | | | JP | 3092070 B2 | 25-09-2000 |
| | | | WO | 8901795 A1 | 09-03-1989 |
| | | | AT | 175270 T | 15-01-1999 |
| | | | CA | 2049337 A1 | 02-11-1990 |
| | | | DE | 69032869 D1 | 11-02-1999 |
| | | | DE | 69032869 T2 | 02-06-1999 |
| | | | DK | 471000 T3 | 30-08-1999 |
| | | | EP | 0471000 A1 | 19-02-1992 |
| | | | JP | 2952037 B2 | 20-09-1999 |
| | | | JP | 5502096 T | 15-04-1993 |
| | | | KR | 166337 B1 | 01-05-1999 |
| | | | WO | 9013795 A2 | 15-11-1990 |
| | | | AT | 177955 T | 15-04-1999 |

For more details about this annex : see Official Journal of the European Patent Office, No. 12/82

## ANNEX TO THE EUROPEAN SEARCH REPORT
## ON EUROPEAN PATENT APPLICATION NO.

EP 01 11 9945

This annex lists the patent family members relating to the patent documents cited in the above-mentioned European search report.
The members are as contained in the European Patent Office EDP file on
The European Patent Office is in no way liable for these particulars which are merely given for the purpose of information.

25-02-2002

| Patent document cited in search report | | Publication date | Patent family member(s) | | Publication date |
|---|---|---|---|---|---|
| US 5349852 | A | | AU | 672154 B2 | 19-09-1996 |
| | | | AU | 1229795 A | 11-05-1995 |
| | | | AU | 658825 B2 | 04-05-1995 |
| | | | AU | 7885891 A | 10-12-1991 |
| | | | BR | 9106446 A | 18-05-1993 |
| | | | CA | 2082057 A1 | 16-11-1991 |
| | | | DE | 69131043 D1 | 29-04-1999 |
| | | | DE | 69131043 T2 | 18-11-1999 |
| | | | EP | 0528947 A1 | 03-03-1993 |
| | | | FI | 925177 A | 13-11-1992 |
| | | | FI | 20001365 A | 08-06-2000 |
| | | | IL | 98152 A | 30-05-1994 |
| | | | IL | 104253 A | 30-03-1995 |
| GB 727891 | A | 13-04-1955 | NONE | | |
| US 3981176 | A | 21-09-1976 | US | B506744 I5 | 13-01-1976 |
| JP 56140249 | A | 02-11-1981 | JP | 1398611 C | 07-09-1987 |
| | | | JP | 62007506 B | 17-02-1987 |
| DE 3125078 | A | 13-01-1983 | DE | 3125078 A1 | 13-01-1983 |
| GB 2087559 | A | 26-05-1982 | NONE | | |
| US 3357245 | A | 12-12-1967 | NONE | | |
| EP 0486264 | A | 20-05-1992 | CA | 2053948 A1 | 15-05-1992 |
| | | | EP | 0486264 A2 | 20-05-1992 |
| | | | JP | 4268416 A | 24-09-1992 |
| GB 574819 | A | 22-01-1946 | NONE | | |

EPO FORM P0459